Europäisches Patentamt

European Patent Office

Office européen des brevets

(19)

(11) Publication number : **0 451 105 A2**

(12) # EUROPEAN PATENT APPLICATION

(21) Application number : 91810221.1

(22) Date of filing : 27.03.91

(51) Int. Cl.[5] : **A61K 9/16, A61K 7/30, A61J 3/10**

(30) Priority : 04.04.90 US 504763

(43) Date of publication of application :
09.10.91 Bulletin 91/41

(84) Designated Contracting States :
BE DE DK ES FR GB GR IT

(71) Applicant : WARNER-LAMBERT COMPANY
201 Tabor Road
Morris Plains New Jersey 07950 (US)

(72) Inventor : Schumacher, Robert William
8 Corwin Street
Kenvil, New Jersey 07847 (US)
Inventor : Mohrle, Raymond L.
11 Hill Road
Denville, New Jersey 07834 (US)

(74) Representative : Silbiger, Jakob, Dr.
c/o CAPSUGEL AG Fabrikmattenweg 2-4
CH-4144 Arlesheim (CH)

(54) Side-by-side effervescent tablet for dental prostheses.

(57)    A multi-component side-by-side effervescent cleansing tablet for dental prostheses is disclosed. The tablet is prepared such that the boundary between the side-by-side components lies essentially vertical.

EP 0 451 105 A2

## FIELD OF THE INVENTION

The present invention relates to a side-by-side effervescent tablet for cleaning and disinfecting dental prostheses. More particularly, this invention is directed to an effervescent denture cleaning tablet having two or more distinct components wherein the boundary between the components lies essentially vertical such that when the tablet is lying horizontally in the vessel, the components are positioned right and left of each other and not above and below each other. The primary advantage of the inventive side-by-side effervescent denture cleaning tablet over a conventional two or more layer denture cleansing tablet is that all components hereof have equal exposure for dissolution when immersed in water regardless of tablet orientation. The side-by-side components may also exhibit differential dissolution rates.

## BACKGROUND OF THE INVENTION

Denture prostheses may be removable (partial or full dentures) or nonremovable (crowns and bridges). These prostheses accumulate plaque, stain and calculus much in the same way as natural teeth and, accordingly, must be frequently cleaned to avoid such problems as malodor, staining, denture stomatitis, inflammatory papillary hyperplasia and chronic candidiasis. Denture cleansers clean by either chemical or abrasive means. The major types of abrasive type cleansers comprise pastes, gels or powders applied by brushing. The chemical cleansers (soaking applications) are primarily peroxides, hypochlorites, persulfates, and dilute acids and bases. Most common are the peroxide cleansers which generally become alkaline solutions of hydrogen peroxides when dissolved in water. The hypochlorites, perborates and persulfates act as cleansers, remove stains, dissolve mucin and are bactericidal and fungicidal. These cleansers can dissolve the organic matrix of plaque but cannot dissolve calculus. The dilute acid cleansers, such as phosphoric acid and hydrochloric acid, can remove stains as well as dissolve inorganic (calcium) phosphate and calculus. There are a wide variety of compositions available to the public including compositions containing abrasive materials for use as scrubbing cleansers as well as chemical compositions without abrasive materials for use as mild surface cleansers such as passive dispersion in a liquid medium, most commonly, water (soaking applications).

U.S. Patent No. 4,417,993 to Gergely teaches a multi-layer effervescent cleansing tablet for dental appliances (soaking application) comprising a faster-dissolving acidic component layer, a slower-dissolving alkaline component layer and an enzyme-containing component layer. The acidic component layer comprises a mixture of sulfamic acid, sodium bicarbonate, sodium carbonate, sodium persulfate, polyethylene glycol and a surface active agent. The alkaline component layer comprises a mixture of sodium carbonate, sodium pyrophosphate, potassium persulfate, a surface active substance and a partial glyceride of a $C_8$ to $C_{12}$ straight-chain vegetable fatty acid. The enzyme component layer comprises a mixture of anhydrous sodium sulfate, polyvinyl pyrrolidone, ascorbic acid, magnesium stearate, and proteolytic enzyme. Each component layer is separately formulated. The acidic component is pre-pressed in a tablet press, the alkaline component is then pressed thereupon and the enzyme component is then pressed onto the alkaline component layer. This disclosure allows an instant acidic cleansing solution which then slowly changes to an alkaline cleansing solution having an enzymatic component.

U.S. Patent 4,256,599 to Krisp et al. discloses a two layer (top and bottom or core-containing flat cylinder) denture cleaning tablet (soaking application) having improved cleansing effect due to the incorporation of sulfamic acid into the first stage layer and the incorporation of ethylenediaminetetra-acetate into both layers of the tablet. The first stage layer is adapted to disintegrate in water prior to the second stage layer and cleanse the denture of proteinaceous substances, fats and bacteria whereas the second layer oxidizes organic compounds and encrustations, softens, emulsifies and forms complexes of the organic agglomerations, and absorbs and oxidizes odorous and discolorant substances.

U.K. Patent Application No. 2 211 460 A to Mizota discloses a method for manufacturing a side-by-side layered tableted type of product. The major components of the product are glucose, sucrose, starch and colorants compressed to produce a two-layered candy.

U.S. Patent 3,997,459 to Bogie et al. is directed to a solid denture cleaning composition (soaking application) having an acidic component, which initially produces an acidic solution, and a polymer-coated neutralizing component, which is released over a period of time to increase the pH of the soaking solution to over 5.5. The composition may also include a bleaching agent.

U.S. Patent No. 3,962,107 to Levin et al. sets forth a water-soluble effervescent layered denture cleanser (soaking application) consisting essentially of a faster-dissolving enzyme layer and a slower-dissolving active oxygen layer. Essential to the enzyme layer are the following: (1) it must be less than one-half of the total thickness of the tablet; and (2) it must have a larger proportion of finely divided powder, such as 30% to 50% of the total weight of the layer, than the proportion of finely divided powder in the active oxygen layer, such as 20% to 30% of the total weight of that layer.

U.S. Patent No. 3,279,995 to Reid teaches a pharmaceutical tablet wherein the surface area remains relatively uniform during dissociation due to the formation of the tablet with specific convexities and concavities.

Thus, although the need for multi-layered denture cleansing tablets (soaking application) and the various problems to be overcome thereby have been known in the art, a single tablet having at least two distinct components wherein both distinct components of the tablet are equally exposed to the environment and wherein differential release of the distinct components may be obtained has been unavailable.

When tablets are placed in solution, they will generally sink to the bottom or float on the surface. Most shaped tablets will inherently orient themselves in solution exhibiting an up side and a down side. Such a tablet having different compositions layered in a top and bottom configuration will unpredictably orient itself in solution to the extent that either layer may be up or down.

The problem associated with two-layer up and down or top and bottom type dental cleansing tablets presents when the tablet is initially immersed in water, that is, it will land with one layer facing up and one layer facing the bottom of the container. The upper layer will tend to disintegrate faster as it is fully exposed to the water whereas the bottom layer will tend to lag behind. This situation would be acceptable if it were known which layer would always land on the top and the product formulated accordingly. However, the face upon which the tablet falls is merely a matter of chance.

A similar phenomenon would occur if the two layer, top-bottom tablet would float. One surface would be out of solution and disintegrate slowly, while the lower surface would be immersed in water and disintegrate more quickly.

U.S. Patent Nos. 4,683,072 and 4,578,207 to Holdt et al. disclose a two-component side-by-side non effervescent toilet bowl cleaning tablet, which adheres to the bowl or urinal where placed, prepared by separately formulating each component and separately extruding each component, then combining the two components into a single composition, the two components occupying different portions of the single composition and cutting the single composition into pieces. The formulations of the two components are such that active chlorine-releasing or active oxygen-releasing disinfectants comprise one of the components and a chlorine- or oxygen-sensitive dye comprises the other component. Alternatively, one of the components can comprise acids or complexing agents to prevent lime, rust or urine deposits and sensitive dyes or fragrances can comprise the other component.

These tablets are for a continuous release of different components and not for a one time differential release which would result in changing the cleaning solution composition as a function of time.

Accordingly, it would be desirable to prepare a two component side-by-side effervescent denture cleansing tablet to insure that all tablet components have equal exposure to the water, regardless of the position of the tablet in the vessel. Moreover, it would also be beneficial if the two or more component side-by-side effervescent tablet could have differential release of the components.

The present invention eliminates the unpredictability of the top and bottom orientation by presenting a side-by-side tablet of two or more components which will inherently position itself in a solution such that tablet orientation will not affect solution contact with the different layers.

SUMMARY OF THE INVENTION

The invention is as described in the claims. In particular the present invention refers to an effervescent cleansing composition in tablet form, the tablet comprising: two or more discrete components which are fixedly attached to each other at a boundary, the boundary lying essentially vertical to the largest face of the tablet such that when the tablet is lying horizontally, components are positioned right and left of each other.

This invention sets forth an effervescent cleansing tablet for dental prostheses, especially dentures. The tablet contains side-by-side components such that the components, upon immersion in an aqueous solution, are equally exposed to such solution. The boundary between the side-by-side components lies essentially vertical such that when the tablet is lying horizontally the side-by-side components are positioned right and left of each other and not above and below (top and bottom) each other. Side-by-side components may also exhibit differential release rates from the time of immersion in an aqueous solution. Release is defined as the disintegration of a component. A component may have more than one ingredient.

BRIEF DESCRIPTION OF THE DRAWINGS

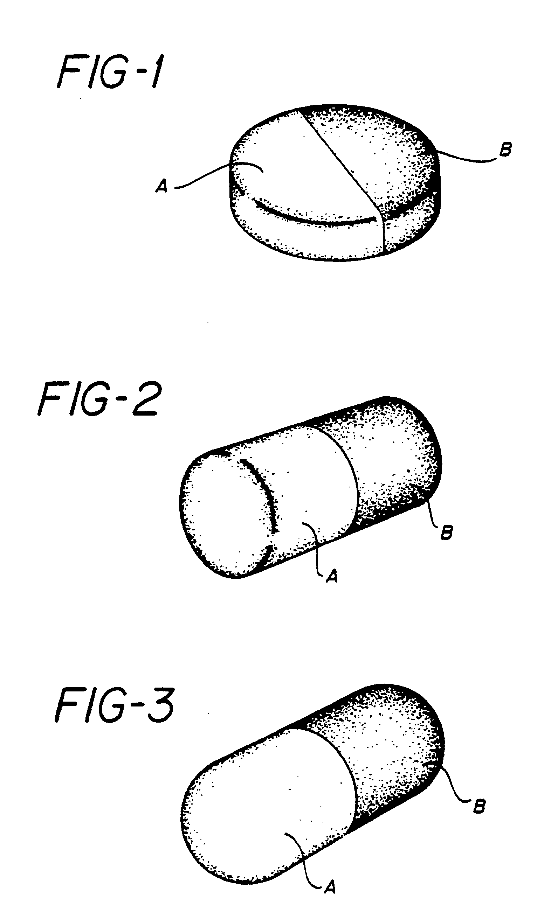

Figure 1 depicts an overview of one embodiment of the inventive side-by-side two component effervescent cleansing tablet for dental prostheses.

Figure 2 depicts an overview of an alternative embodiment of the present invention.

Figure 3 depicts an overview of an alternative embodiment of the present invention.

DETAILED DESCRIPTION OF THE INVENTION

The present invention teaches an effervescent cleansing tablet for dental prostheses, a two or more component side-by-side system. The denture cleansing tablet is comprised of two or more discrete side-by-side components which are fixedly attached to each other at a boundary. The boundary lays essentially vertically between the side-by-side components of the tablet. Side-by-side components are positioned right and left of each other and not above and below (top and bottom) each other. Side-by-side components, being different components, may be formulated to provide differential dissolution rates from the time of immersion in an aqueous solution.

The advantages of the present side-by-side two or more component system are that sequential release of the components may be easily provided for systems having components which are reactive or sensitive to each other while at the same time, providing better cleansing of the dental prostheses with greater control and more uniform results over the prior art layered systems. It is less costly to produce and, also, more convenient to the user than a two tablet two component system where each tablet has a different dissolution rate.

Differential or controlled release would be useful in tablets containing enzymes and bleaching agents; bleaching agents and bleach deactivators; and two or more incompatible bleaching agents. Cleaning systems which benefit from a pH change are another application of the present invention.

The inventive side-by-side component system is prepared from primarily conventional denture cleansing constituents including acidifying agents, alkalizing agents, effervescing agents, oxidizing agents, bleaching agents, chelating agents, surfactant/foaming agents, lubricants, various additives such as colorants and flavorants and various tabletting agents, lubricants, excipients, disintegrants and the like.

Typical bleaching agents include hypochlorite generating agents and peroxide or active oxygen generating agents. The active oxygen agent is preferably one selected from the group consisting of sodium perborate monohydrate, potassium persulfate, sodium carbonate, peroxide, diperisophthalic acid and potassium peroxydiphosphate. The effervescence producing composition may also contain a bleaching agent which may be an oxygenating bleach such as Oxone (Trade Mark of Du Pont Company), whose active constituent is potassium monopersulphate; perborate salts or percarbonate salts.

As used in the specification and claims, the term "hypochlorite generating agent" means any compound which upon contact with water generates or releases hypochlorite ion directly or by the interaction of two or more compounds. Illustrative, non-limiting examples of the hypochlorite generating agent of this invention are heterocyclic N-chloroimides such as chloroisocyanurate, e.g. sodium dichloroisocyanurate, potassium dichloroisocyanurate, or trichlorocyanuric acid; complex salts of two or more compounds, e.g., [(mono-trichloro)-tetra-(monopotassiumdichloro)] pentaisocyanurate; other N-chloroimides, e.g., sodium p-toluene-sulfonochloramide, N,N-dichloro-p-toluenesulfonamide, sodium benzene-sulfonchloramide, N,N-dichlorobenzene-sulfonamide, N-chlorosuccinate. Still other compounds which liberate hypochlorite/ chlorine on contact with water are N-chloro malonimide, N-chloro phthalinide and N-chloro naphthalimide; the hydantoins such as 1,3-dichloro-5,5-dimethylhydantoin; N-monochloro-C,C-dimethylhydantoin, methylene-bis-(N-chloro-C,C-dimethylhydantoin); 1,3-dichloro-5-ethylhydantoin and 1,3-dichloro-5,5-diisoethylhydantoin; dry solid inorganic compound such as lithium hypochlorite, calcium hypochlorite; mixtures of salts such as sodium persulfate and sodium chloride; sodium perborate and chlorinated 5-triazine triones.

Acidifying agents are utilized to remove stains as well as dissolve inorganic (calcium) phosphates and calculus. Typical acidifying agents include citric acid, malic acid, phosphates, bisulfate, sulfamic acid, pyrophosphates, and tartaric acid in amounts ranging from about 1% to about 65%.

Alkalizing agents are useful in removing soil and stains while providing a reduction in the soaking time necessary to cleanse the dentures. Typical alkalizing agents include alkali metal and alkaline earth metal carbonates, tetrapyrophosphates, tripolyphosphates, borates, phosphates, metasilicates, hydroxides and mixtures thereof including sodium carbonate, sodium metasilicate, borax, sodium pyrophosphates, and sodium hydroxide in amounts ranging from about 1% to about 65%.

The effervescing agents provide mechanical agitation of the cleansing solution thus enhancing the cleansing effects of the other constituents. These systems generate gases such as oxygen, carbon dioxide and nitrogen which stir the solution thereby providing fresh cleansing solution to the surface of the prostheses. Such gas producing systems are known in the art. Typical effervescent agents include carbonates and peroxide systems. The term "effervescent agent" means a compound utilized to cause the denture cleaning composition to effervesce, resulting in disintegration of the tablet and stirring of the cleansing solution. Illustrative, non-limiting examples of effervescent agents are anhydrous sodium perborate; sodium perborate monohydrate; a mixture of an alkali metal bicarbonate and a water soluble organic acid, e.g., sodium bicarbonate, ammonium bicarbonate, potassium bicarbonate with citric acid, ascorbic acid and tartaric acid. Typical effervescent systems may comprise 5% to 85% of the denture cleanser.

A chelating agent (sequesterant) may be used to maintain solution clarity, break up calculus, and control heavy metals. Useful chelatng agents include ethylenediaminetetraacetic acid (EDTA) and its alkali salts as well as other polyfunctional organic acids such as citric acid, maleic acid and their salts. These agents may be present in amounts ranging from about 0.5% to about 50%.

The present compositions may optionally contain sequestrants for the purpose of maintaining solution clarity, in the instance where the compositions are placed in solution. The sequestrants may also assist in the inhibition of corrosion and tarnish of articles soaked in solutions containing the present compositions. Useful sequestrants include ethylene-diaminetetraceticacid (EDTA) and its corresponding alkali salts, as well as other polyfunctional organic acids, such as citric acid, maleic acid and their corresponding salts.

Those chelating or sequestering agents which are to be used in amounts greater than about 10% up to about 50% by weight are phosphonic acid and its derivatives and carboxylic acid derivatives. Among those specific phosphonic acid derivatives are the salts of ethane-1-hydroxy-1,1-diphosphonic acid. For example, aminotri (methylene phosphonic acid), 1-hydroxyethylidene-1,1-diphosphonic acid, ethylenediamine tetra (methylene phosphonic acid), ethylenediaminetetra (methylene phosphonic acid), hexamethylenediaminetetra (methylene phosphonic acid), diethylene triamine penta (methylene phosphonic acid), among others. The alkali metal salts and analogues of the above phosphonates are also useful.

Surfactant/foaming agents may be used to assist in maintaining the foaming action of the aqueous solution and to aid in cleansing. Such agents are organic and inorganic detergents, including non-ionic detergents such as polyoxyethylene ethers of aromatic and aliphatic alcohols as well as polyoxyethylene ethers of hydrophobic propylene oxide polymers; cationic detergents such as alkyl amine oxides, fatty acid amines and fatty acid amides and anionic detergents such as alkyl/aryl sulfates, alkyl/aryl sulfonates and alkyl/aryl sulfoacetates. These agents may be included in amounts ranging from about 0.1% to about 5%.

When a pH change is desired, the compositions of the present invention may contain an acidic component and basic or neutralizing component. Preferably the acidic solution formed when the cleaning composition is mixed with water has a pH of less than 2.5, preferably less than 2, and the solution remaining when all the neutralizing component has been released has a pH greater than 5.5.

Suitable acidic components include acids such as sulphamic acid, acetodiphosphonic acid, solid organic acids such as citric acid and tartaric acid or acid salts such as sodium bisulphate.

Preferably, the neutralizing component contains a carbonate salt such as sodium carbonate or a bicarbonate salt such as sodium bicarbonate or a mixture thereof so that turbulence is caused by the evolution of carbon dioxide gas which occurs during the neutralization of the acidic solution formed when the composition has been added to water. Additionally, a phosphate salt such as trisodium phosphate or a hydroxide such as calcium hydroxide may be included in the neutralizing component.

Tablet lubricants may be included for their traditional purposes. Typical lubricants include stearates, waxes, and polytetrafluoroethylene in amounts ranging from about 1% to about 6%.

Flavorants may be added in various amounts, depending upon choice. Varieties of mint are preferred and may be included and blended in various combinations by one having ordinary skill in the art.

Colorants useful herein are those known as F.D. & C. and D. & C. dyes and lakes. These materials are certified by the Federal Food and Drug Administration as being acceptable for use in food, drug and cosmetic applications and drug and cosmetic colorings. The materials useful for denture cleansers are typically water-soluble and include indigo dye (F.D. & C. Blue No. 2), as well as F.D. & C. Green No. 3, F.D. & C. blue 1 and F.D. & C. yellow 5. Colorants may be present in amounts ranging from about 0.05% to about 1%.

The present invention is prepared by selecting the appropriate constituents for each component and separately preparing each component in any manner known to one having ordinary skill in the art. The inventive side-by-side cleanser is then prepared by initially charging one of the components into one of the sections of a die cavity having therein a partition or partitions which divide the die cavity into two or more discrete side-by-side sections. The other component(s) may then be charged in a like manner into the other section(s) of the die cavity and the partition(s) removed. The charged die cavities are then pressed by conventional means to provide the desired tablet.

In a preferred embodiment, a process for preparing a side-by-side effervescent cleansing composition tablet of two or more components comprises:

(a) separately mixing until homogenous the constituents of each component;

(b) charging one of the components into one section of a die cavity having therein a number of partioned sections equivalent to the number of components;

(c) charging the other components into the other sections of the die cavity;

(d) removing the partitions;

5

(e) pressing the die cavity to form a compressed tablet therein; and
(f) removing the tablet from the die cavity, wherein the number of partitioned sections is equivalent to the number of components.

EXAMPLES 1-4

Component A

The following powders were blended together for about 2 to 3 minutes until a homogenous mixture was obtained:

| Constituent | Percent w/w |
|---|---|
| sodium metasilicate | 12.45 |
| EDTA tetrasodium dihydrate | 3.73 |
| sodium dichloroisocyanurate | 53.30 |
| peppermint flavor | 3.11 |
| sodium perborate monohydrate | 26.10 |
| surfactant | .94 |
| polytetrafluoroethylene | .37 |
| | 100.00 |

Then, 15.0 grams of the above mixture was combined with 6.0 grams of citric acid until homogeneous.

Component B

The following powders were blended together for about 2 to 3 minutes until a homogenous mixture was obtained:

| Constituent | Percent W/W |
|---|---|
| sodium bicarbonate | 11 |
| coloring agent | 0.16 |
| EDTA tetrasodium dihydrate | 4 |
| sodium tripolyphosphate | 10 |
| sodium carbonate | 9 |
| citric acid | 4 |
| sodium sulfate | 5 |
| potassium monopersulfate | 38.62 |
| sodium perborate monohydrate | 12 |
| sodium perborate anhydrous | 3 |
| flavo | 1 |
| surfactant | 0.6 |
| sodium benzoate | 1.5 |
| magnesium stearate | 0.03 |
| polytetrafluoroethylene | 0.09 |
| | 100.00 |

Conventional single component tablets were also prepared from each of components A and B. Single component A tablets were prepared from 2.1 grams of the mixture pressed at 5000 lbs for 10 sec. Single component B tablets were prepared from 1.5 grams of the mixture pressed at 5000 lbs for 10 sec.

Conventional two layer (top and bottom) tablets were prepared by charging 2.1 grams of component A into the die cavity of a tablet press and leveled. Then 1.5 grams of component B was charged immediately on top of component A and leveled. The tablets were then pressed at 5000 lbs for 10 seconds.

Inventive side-by-side tablets were prepared by using a partition to separate the die cavity into two side-by-side compartments. One compartment was charged with 2.1 grams of component A and the other compartment was charged with 1.5 grams of component B. The partition was gently removed so as to not mix the powders and tablets were then pressed at 5000 lbs. for 10 seconds.

Dissolution studies were carried out on the conventional two layer (top and bottom) tablets, the inventive side-by-side tablets and the conventional single component tablets by immersing the tablets in 125 mil of about 44° to 46°C water. Three trials of each tablet type were carried out and the following results were obtained.

| Tablet | Dissolution time |
|---|---|
| single component A | 1 to 1 1/2 minutes |
| single component B | 4 minutes |
| conventional two layer | |
| (1) component A up on immersion | component A - 1 1/2 min. component B - 3 min. |
| (2) component B up on immersion | component A - 2 1/2 min. component B - 2 1/2 min. |
| inventive side-by side | component A - 1 1/2 min. component B - 4 min. |

These studies demonstrate that the inventive side-by-side tablet eliminates the chance-influenced performance of the conventional layered (top and bottom) tablet and that the inventive side-by-side tablet acts as if it was two conventional single component tablets immersed in water simultaneously.

The invention as thus hereinbefore described may obviously be varied in many ways. Such variations are not to be regarded as a departure from the spirit and scope of the invention and all such modifications are intended to be included within the scope of the following claims.

## Claims

1. An effervescent cleansing composition in tablet form, the tablet comprising: two or more discrete components which are fixedly attached to each other at a boundary, the boundary lying essentially vertical to the largest face of the tablet such that when the tablet is lying horizontally, components are positioned right and left of each other.

2. The effervescent cleansing composition in tablet form of claim 1, wherein the components are differentially released when the composition is immersed in water.

3. The effervescent cleansing composition in tablet form of claim 1, wherein the tablet is concave shaped.

4. The effervescent cleansing composition in tablet form of claim 1, wherein the tablet is convex shaped.

5. The effervescent cleansing composition in tablet form of claim 1, wherein the tablet is disk or modified disk shaped.

6. The effervescent cleansing composition in tablet form of claim 1, wherein the tablet is cylindrically shaped.

7. The effervescent cleansing composition in tablet form of claim 1, wherein the tablet is polyhedral shaped.

8. The effervescent cleansing composition in tablet form according to anyone of the claims 1 to 7, wherein
   (a) one component is comprised of an alkalizing agent, buffering agents, an active chlorine compound, a flavoring agent, a peroxide source, a surfactant and a lubricant, and
   (b) a second component is comprised of an acidifying agent, buffering agents, a chelating agent, an active chlorine compound, a flavoring agent, a peroxide source, a surfactant and a lubricant.

9. The effervescent cleansing composition in tablet form according to anyone of the claims 1 to 7, wherein

(a) one component is comprised of a peroxide source, buffering agents, a carbonating agent, a flavoring agent, a surfactant and a lubricant, and

(b) a second component is comprised of an active chlorine compound, buffering agents, a carbonating agent, a chelating agent, a flavoring agent, a surfactant and a lubricant.

10. The effervescent cleansing compositing in tablet form according to anyone of the claims 1 to 7, wherein

(a) one component is comprised of an enzyme, buffering agents, a carbonating agent, a flavoring agent, a surfactant and a lubricant, and

(b) a second component is comprised of a bleaching agent, buffering agents, a carbonating agent, a flavoring agent, a surfactant, a chelating agent and a lubricant.

11. A process for preparing a side-by-side effervescent cleansing composition of two or more components as claimed in anyone of the claims 1 to 10 comprising:

(a) separately mixing until homogenous the constituents of each component,

(b) charging one of the components into one section of a die cavity having therein a number of partioned sections equivalent to the number of components.

(c) charging the other components into the other sections of the die cavity,

(d) removing the partitions,

(e) pressing the die cavity to form a compressed tablet therein, and

(f) removing the tablet from the die cavity, wherein the number of partitioned sections is equivalent to the number of components.

*FIG-1*

*FIG-2*

*FIG-3*